Europäisches Patentamt

European Patent Office

Office européen des brevets

(11) Veröffentlichungsnummer: **0 208 914 B1**

## EUROPÄISCHE PATENTSCHRIFT

(45) Veröffentlichungstag der Patentschrift: **29.07.92**

(51) Int. Cl.⁵: **G01N 37/00**, G01N 33/00

(21) Anmeldenummer: **86107997.8**

(22) Anmeldetag: **12.06.86**

(54) **Vorrichtung zur Erzeugung eines definierten Gas/Staub-Gemisches.**

(30) Priorität: **17.06.85 LU 85955**

(43) Veröffentlichungstag der Anmeldung:
**21.01.87 Patentblatt 87/04**

(45) Bekanntmachung des Hinweises auf die
Patenterteilung:
**29.07.92 Patentblatt 92/31**

(84) Benannte Vertragsstaaten:
**BE DE FR GB IT LU NL**

(56) Entgegenhaltungen:
**EP-A- 0 100 281**

(73) Patentinhaber: **EUROPÄISCHE ATOMGEMEIN-
SCHAFT (EURATOM)
Bâtiment Jean Monnet Plateau du Kirchberg
Boîte Postale 1907
L-1019 Luxembourg(LU)**

(72) Erfinder: **Pickering, Stephen
Schubertstrasse 12a
W-7514 Eggenstein-Leopoldshafen(DE)**

(74) Vertreter: **Weinmiller, Jürgen
Lennéstrasse 9 Postfach 24
W-8133 Feldafing(DE)**

**Beschreibung**

Die Erfindung bezieht sich auf eine Vorrichtung zur Erzeugung eines definierten Gemisches aus einem Gas und Keramikstaub, mit einem von dem Gas durchströmten Behälter, in dem sich das Keramikmaterial befindet.

Keramikstaub-/Gasgemische spielen bei der Analyse der Staubbelastung der Umwelt eine große Rolle. Um die Staubbelastung der Umwelt messen zu können, braucht man Meßgeräte, die anhand von definierten Gemischen geeicht werden. Die Erfindung betrifft daher eine Vorrichtung, mit der solche definierten Gemische hergestellt werden können.

Es liegt nahe, zu diesem Zweck ein Pulver einer gegebenen Granulometrie von einem Nebulisator in den Gasstrom einzustreuen. Die damit erreichte Verteilung von Staub im Gemisch ist jedoch besonders bei Konzentrationsraten, wie sie in der Umwelt vorkommen, über die Dauer eines Versuchs hinweg, d.h. in der Größenordnung von einer Stunde und mehr, nicht hinreichend konstant.

Aufgabe der Erfindung ist es daher, eine Vorrichtung zur Erzeugung eines definierten Gemisches aus einem Gas und Keramikstaub anzugeben, die bei verhältnismäßig großer Staubkonzentration über längere Zeit (in der Größenordnung einer Stunde und mehr) eine konstante Konzentrationrate ergibt.

Diese Aufgabe wird durch die Vorrichtung, wie sie im Anspruch 1 gekennzeichnet, ist, gelöst. Bezüglich von Merkmalen eines bevorzugten Ausführungsbeispiels der Erfindung wird auf den Unteranspruch verwiesen.

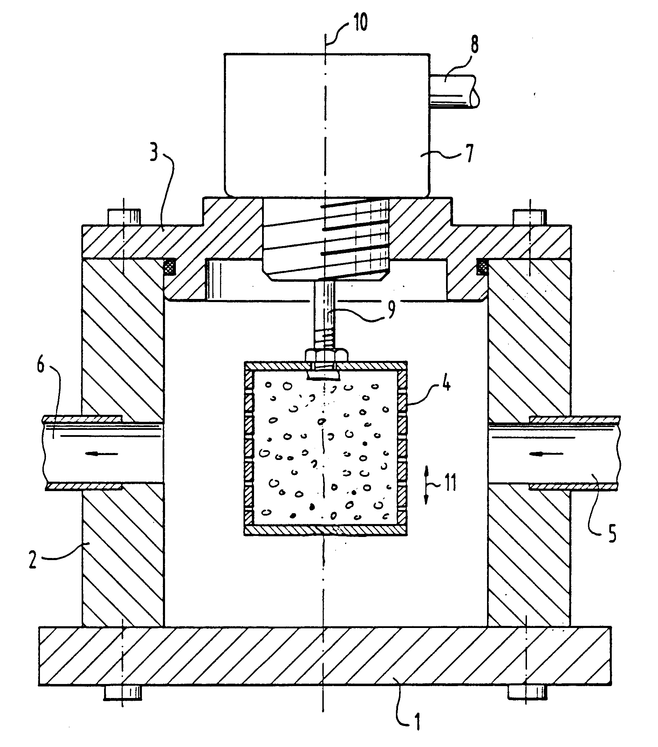

Die Erfindung wird nun anhand eines bevorzugten Ausführungsbeispiels näher erläutert, das in der einzigen Figur im Schnitt zu sehen ist.

Die Vorrichtung besteht im wesentlichen aus einem zylindrischen Behälter aus einer Al-Legierung mit einem Boden 1, mit einer zylindrischen Seitenwand 2 und mit einem Deckel 3, sowie aus einem Käfig 4, in dem sich Sinterkörner aus dem gewünschten Metalloxid, z.B. $Al_2O_3$, befinden. Der Boden 1, die Seitenwand 2 und der Deckel 3 sind miteinander fest verschraubt. In der zylindrischen Seitenwand sind einander diametral gegenüber ein Gaseinlaß 5 und ein Gasauslaß 6 vorgesehen. Im Deckel 3 ist axial ein Vibrator 7 eingeschraubt, der über ein Kabel 8 mit elektrischer Energie versorgt wird. Eine Vibrationsachse 9 des Vibrators ragt axial in den Behälter hinein und trägt den Käfig 4. Die Vibrationsrichtung verläuft in Richtung der Zylinderachse der Vorrichtung 10, wie durch den Pfeil 11 angedeutet ist, wahrend die Richtung der Gasströmung waagerecht verläuft.

Der Käfig 4 wird von der Vibrationsachse 9 axial getragen und ist auf dieser festgeschraubt. Auch dieser Käfig hat zylindrische Form und besteht aus einer Aluminiumlegierung. Die zylindrische Mantelfläche ist mit einer Vielzahl von Löchern versehen, durch die Staub austreten kann, nicht aber die Sinterkörner, da deren Durchmesser deutlich größer als der der Löcher ist. Der zylindrische Käfigbereich liegt etwa in Flucht zu den Gaseinlaß- und -auslaßöffnungen 5 und 6, so daß Staubpartikel, die sich bei der Vibration im Käfig bilden, durch den Gasstrom mitgerissen werden.

Es hat sich überraschend gezeigt, daß der Abrieb an Käfigmaterial sich praktisch nicht bemerkbar macht. Offenbar weichen die Käfigwände aufgrund ihrer elastischen und plastischen Eigenschaften den Stößen der Sinterkörner aus, so daß derselbe Käfig für die Verstäubung der unterschiedlichsten Sintermaterialien verwendet werden kann.

Die nachstehende Tabelle enthält Betriebswerte der erläuterten Vorrichtung unter folgenden Bedingungen : Es wurden 3,5 g Aluminiumoxid-Sinterkörner in den Käfig 4 eingefüllt. Als Gas wurde Luft mit einem Durchsatz von 1 l pro Minute verwendet. Der Vibrator wurde mit normalen Wechselstrom gespeist, und das Staubgemisch wurde mit einem Granulometer gemessen. In der ersten Spalte sind die Meßzeitpunkte vom Beginn des Versuchs aus gerechnet aufgeführt. Nach zweieinhalb Stunden wurde der Versuch abgebrochen, da neue Sinterkörner eingefüllt werden mußten.

Während dieser ganzen Zeit unterlag die zahlenmäßige Konzentration von Staubteilchen in der Luft kaum Schwankungen, wie sich aus der zweiten Spalte dieser Tabelle ergibt. Die angegebenen Zahlenwerte stellen die Anzahl der Staubteilchen in einem Milliliter dar. Die Meßwerte schwanken um einen Mittelwert von 9188 Partikel mit einer Schwankungsbreite von ± 3, und zwar ohne einheitliche Tendenz über die Dauer des Versuchs.

Die nächste Spalte gibt Meßwerte für die Massenkonzentration in Milligramm pro $m^3$ an. Hier erkennt man, daß diese Konzentration stetig abnimmt, da zunehmend Teilchen kleineren Durchmessers abgeschieden werden.

Die nächste Spalte gibt den mittleren aerodynamischen Durchmesser der Teilchen in Mikrometer, bezogen auf die Teilchenzahl, und die fünfte Spalte den mittleren aerodynamischen Durchmesser in Mikrometer bezogen auf die Masse an. Diese Spalten bestätigen die obige Erklärung für die praktisch über die ganze Versuchsdauer ohne einheitliche Veränderungstendenz konstant bleibende Konzentration der

2

Teilchenzahl in dem Gemisch (Spalte 2).

Es ist bemerkenswert, daß die Endwerte nach zweieinhalb Stunden mit einer Restfüllung an Sinterkörnern von nur noch 0,3 g im Käfig erreicht wurden. Diese Ergebnisse erscheinen überraschend, insbesondere im Vergleich mit der nur sehr kurzfristigen Konstanz der Konzentration, die man mit einem Nebulisator erreichen kann.

| Konzentration | | | | |
|---|---|---|---|---|
| Zeit (min) | Anzahl pro ml | (mg/m³) | $\phi$Zahl ($\mu$m) | $\phi$Masse ($\mu$m) |
| 1 | 9398 | 111.7 | 1.87 | 5.73 |
| 5 | 9217 | 111.4 | 1.90 | 5.75 |
| 10 | 9173 | 107.0 | 1.80 | 5.70 |
| 15 | 9308 | 110.4 | 1.88 | 5.70 |
| 20 | 9164 | 110.9 | 1.88 | 5.78 |
| 30 | 9107 | 107.3 | 1.85 | 5.75 |
| 40 | 9875 | 99.13 | 1.83 | 5.61 |
| 60 | 9000 | 99.86 | 1.82 | 5.64 |
| 75 | 8870 | 93.10 | 1.80 | 5.60 |
| 100 | 9176 | 92.99 | 1.77 | 5.60 |
| 110 | 9003 | 88.12 | 1.77 | 5.50 |
| 120 | 9317 | 85.48 | 1.74 | 5.48 |
| 130 | 9505 | 89.19 | 1.75 | 5.50 |
| 140 | 9357 | 82.42 | 1.72 | 5.42 |
| 150 | 9486 | 80.54 | 1.71 | 5.40 |

Die Erfindung ist nicht auf das im einzelnen dargestellte Ausführungsbeispiel beschränkt. So könnte der Behälter auch anstelle eines Kreisquerschnitts einen quadratischen Querschnitt besitzen. Die Schüttelachse könnte auch waagerecht liegen, während das Gas senkrecht durch den Behälter geleitet wird.

**Patentansprüche**

1. Vorrichtung zur Erzeugung eines definierten Gemisches aus einem Gas und Keramikstaub, mit einem von dem Gas durchströmten Behälter, in dem sich das Keramikmaterial befindet, dadurch **gekennzeichnet,** daß das Keramikmaterial sich in Form von Sinterkörnern oder -brocken in einem Metallkäfig (4) befindet, der an einem Vibrator (7) befestigt frei in den Behälter (1, 2, 3) hineinragt, wobei die Maschenweite des Käfigs deutlich kleiner als der Durchmesser der Sinterkörper und deutlich größer als die Durchmesser der Staubpartikel gewählt ist.

2. Vorrichtung nach Anspruch 1, dadurch gekennzeichnet, daß der Behälter (1, 2, 3) zylindrisch geformt ist und aufrecht steht, daß das Gas waagerecht und diametral den Behälter durchströmt und daß der Vibrator (7) in einem Zylinderdeckel (3) axial derart befestigt ist, daß der Käfig (4) in den Gasstrom hineinragt.

**Claims**

1. A device for producing a defined mixture of a gas and ceramic dust, with a container which contains the ceramic material and is traversed by the gas, characterized in that the ceramic material is presented in the shape of sinter grains or sinter lumps in a metal cage (4) which protrudes into the container (1, 2, 3) and is fixed to a vibrator (7), the mesh width of the cage being chosen significantly smaller than the diameter of the sinter bodies and significantly larger than the diameters of the dust particles.

2. A device according to claim 1, characterized in that the container (1, 2, 3) is cylinder-shaped and stands upright, that the gas flows through the container horizontally and diametrically and that the vibrator (7) is secured axially in a cylinder lid (3) in such a way that the cage (4) intersects the gas flow.

**Revendications**

1. Dispositif pour la production d'un mélange défini d'un gaz et de poussière céramique, avec un récipient qui contient le matériau céramique et qui est traversé par le gaz, caractérisé en ce que le matériau céramique se trouve en forme de grains ou de morceaux frittés dans une cage métallique (4), qui est fixée à un vibrateur (7) et fait saillie librement dans le récipient (1, 2, 3), la largeur des mailles de la cage étant sensiblement plus petite que le diamètre des corps frittés et sensiblement plus grande que les diamètres des particules de poussière.

2. Dispositif selon la revendication 1, caractérisé en ce que le récipient (1, 2, 3) est cylindrique et est disposé debout, que le gaz le traverse horizontalement et diamétralement et que le vibrateur (7) est fixé axialement sur un couvercle du cylindre (3) de telle façon que la cage (4) intersecte le courant de gaz.